# FASCICULE DE BREVET EUROPEEN

(11) **EP 1 054 668 B1**
(45) Date de publication et mention de la délivrance du brevet: **31.08.2005**
(21) Numéro de dépôt: 99903773.2
(22) Date de dépôt: 16.02.1999
(51) Int. Cl.: A61K 31/425, A61K 31/428, A61P 27/06, A61K 31/426, A61P 27/00

(54) **UTILISATION DU RILUZOLE DANS LE TRAITEMENT DE L'ISCHEMIE RETINIENNE**
VERWENDUNG VON RILUZOL ZUR BEHANDLUNG DER RETINALISCHEMIE
USE OF RILUZOLE IN THE TREATMENT OF RETINAL ISCHEMIA

(30) Priorité: 17.02.1998 FR 9801915
(43) Date de publication de la demande: 29.11.2000
(73) Titulaire: CENTRE NATIONAL DE LA RECHERCHE SCIENTIFIQUE (CNRS), 75794 Paris Cedex 16 (FR)
(72) Inventeur: LAZDUNSKI, Michel, F-06000 Nice (FR); HEURTEAUX, Catherine, F-06600 Antibes (FR)
(74) Mandataire: Breese, Pierre
(86) Numéro de dépôt international: PCT/FR1999/000350
(87) Numéro de publication internationale: WO 1999/042103

(56) Documents cités:
- WO-A-94/20103
- WO-A-95/19170
- CHEW S.J. ET AL: "Neuroprotection: The next breakthrough in glaucoma ? Proceedings of The Third Annual Optic Nerve Rescue and Restoration Think Tank." JOURNAL OF GLAUCOMA, (1997) 6/4 (263-266). REFS: 0 ISSN: 1057-0829 CODEN: JOGLES, XP002075483 United States
- DREYER E B: "Intelligent selection of glutamate antagonists for the management of glaucoma." ANNUAL MEETING OF THE ASSOCIATION FOR RESEARCH IN VISION AND OPHTHALMOLOGY, PARTS 1-2, FORT LAUDERDALE, FLORIDA, USA, MAY 11-16, 1997. INVESTIGATIVE OPHTHALMOLOGY & VISUAL SCIENCE 38 (4 PART 1-2). 1997. S221. ISSN: 0146-0404, XP002075484
- CAPRIOLI J.: "Neuroprotection of the optic nerve in glaucoma." ACTA OPHTHALMOLOGICA SCANDINAVICA, (1997) 75/4 (364-367). REFS: 42 ISSN: 1395-3907 CODEN: AOSCFV, XP002075485 Denmark
- LOMBARDI G ET AL: "Glutamate receptor antagonists protect against ischemia -induced retinal damage." EUROPEAN JOURNAL OF PHARMACOLOGY, (1994 DEC 27) 271 (2-3) 489-95. JOURNAL CODE: EN6. ISSN: 0014-2999., XP002075486 Netherlands
- IVERSEN L L: "Pharmacological approaches to the treatment of ischaemic neuronal damage." EYE, (1991) 5 ( PT 2) 193-7. REF: 21 JOURNAL CODE: EYE. ISSN: 0950-222X., XP002075487 ENGLAND: United Kingdom
- MOSINGER J L ET AL: "Blockade of both NMDA and non-NMDA receptors is required for optimal protection against ischemic neuronal degeneration in the in vivo adult mammalian retina." EXPERIMENTAL NEUROLOGY, (1991 JUL) 113 (1) 10-7. JOURNAL CODE: EQF. ISSN: 0014-4886., XP002075488 United States
- OSBORNE N N ET AL: "Antigens associated with specific retinal cells are affected by ischaemia caused by raised intraocular pressure: effect of glutamate antagonists." NEUROCHEMISTRY INTERNATIONAL, (1996 SEP) 29 (3) 263-70. JOURNAL CODE: BNU. ISSN: 0197-0186., XP002075489 ENGLAND: United Kingdom
- SUGAWARA T ET AL: "Protective effect of dextromethorphan on the ischemic retinal damage in rabbit." NIPPON GANKA GAKKAI ZASSHI. ACTA SOCIETATIS OPHTHALMOLOGICAE JAPONICAE, (1992 JAN) 96 (1) 90-5. JOURNAL CODE: 22O. ISSN: 0029-0203., XP002075490 Japan

## Description

La présente invention concerne l'utilisation d'inhibiteur de la libération du glutamate dans le traitement de l'ischémie rétinienne. L'invention concerne plus particulièrement l'application du riluzol et de ses dérivés actifs en neuroprotection, ou les sels pharmaceutiquement acceptables de ces composés, à la préparation de médicaments destinés au traitement et/ou à la prévention des maladies associées à un ischémie rétinienne.

L'ischémie rétinienne s'observe en clinique dans des situations aiguës telles que les occlusions artérielles ou veineuses de la rétine, les contusions oculaires et aussi dans des pathologies chroniques telles que la dégénérescence maculaire liée à l'âge (DMLA), le glaucome, la rétinopathie diabétique, la rétinopathie du prématuré, les maladies inflammatoires et les hémopathies, qui conduisent à un dommage rétinien aboutissant même, dans nombre de cas, à une dégénérescence totale de la rétine. Le glaucome et la dégénérescence maculaire liée à l'âge sont devenus les principales causes de la malvoyance dans les pays occidentaux, liées à l'augmentation de l'espérance de vie, et constituent un véritable problème de santé publique. Ainsi, de part la fréquence et la sévérité de l'atteinte neuronale rencontrée dans ces affections oculaires, le concept de neuroprotection suscite un grand intérêt en ophtalmologie.

L'ischémie est un élément essentiel dans la pathogénie de ces atteintes rétiniennes. C'est aussi un phénomène éminemment complexe, car multifactoriel. L'analyse du dommage rétinien permet d'accéder à la recherche d'une prévention de la neurodégénérescence. Autant, les mécanismes impliqués dans la dégénérescence neuronale au niveau cérébral ont été largement étudiés, autant il existe actuellement peu de données sur la neurodégénérescence induite par ischémie rétinienne.

Il n'existe à ce jour aucune thérapie efficace. Des progrès réels ont été faits dans la compréhension des causes de la mort neuronale dans le système nerveux central et les mêmes concepts ont été récemment appliqués à la rétine. Le glutamate est le neurotransmetteur excitotoxique majeur dans l'ischémie cérébrale (7). L'excitotoxicité, liée à la libération accrue du glutamate (7, 18) apparaît comme une des causes principales conduisant à la dégénérescence neuronale. La neurotoxicité du glutamate endogène a été impliquée dans plusieurs maladies neuro-dégénératives, traumatiques, ischémiques du système nerveux central (7, 9). Le glutamate joue un rôle important dans la transmission synaptique rétinienne (17), aussi l'hypothèse excitotoxique (7, 18) pourrait être utilisée de la même façon dans l'ischémie rétinienne. L'accumulation extracellulaire accrue du glutamate, qui se produit suite à une ischémie et à une hypoxie, induirait une dégénérescence rétinienne. Le déficit énergétique résultant inhiberait la capacité des fibres de Müller à recapter le glutamate libéré. Et l'accumulation du neurotransmetteur dans les espaces extracellulaires provoquerait une dépolarisation des cellules ganglionnaires et une augmentation du calcium intracellulaire, aboutissant à la mort cellulaire. Lucas et Newhouse (13) furent les premiers à montrer la destruction des couches internes de la rétine suite à l'injection systémique de glutamate. De faibles doses de glutamate administrées au long cours, dans la cavité vitréenne du rat, se révèlent être toxiques pour les cellules ganglionnaires de la rétine (21). Une libération spécifique du glutamate suite à une ischémie induite par hyper-pression intraoculaire a été décrite chez le lapin (12) et une augmentation significative de glutamate a été trouvée dans la cavité vitréenne de l'homme et du singe atteints de glaucome (8).

Le riluzole, aussi désigné 2-amino-6-trifluoromethoxy benzothiazole, est un composé connus pour ses nombreuses propriétés. Il est décrit comme anticonvulsivant et antiépileptique (brevet Européen No. 50 551), dans le traitement des lésions neurologiques liées à des traumastismes (brevet Français No. 2 699 077), dans le traitement de la schizophrénie (brevet Europen No. 305 276) des troubles du sommeil et de la dépression (brevet Européen No. 305 277). Plus récemment, l'utilisation du riluzole a été proposé dans le traitement des maladies mitochondriales (demande de brevet internationale No. WO95/19170).

Il a maintenant été trouvé que le riluzole, et ses dérivés actifs en neuroprotection décrits dans l'art antérieur, peut être utilisé dans le traiment ou la prévention des maladies associées à une ischémie rétinienne, comme les occlusions artérielles ou veineuses de la rétine, les contusions oculaires et aussi dans des pathologies chroniques telles que la dégénérescence maculaire liée à l'âge (DMLA), le glaucome, la rétinopathie diabétique, la rétinopathie du prématuré, les maladies inflammatoires et les hémopathies.

Le riluzole présente la propriété d'inhiber la libération présynaptique du glutamate (6, 10, 15). Les propriétés neuroprotectrices du riluzole ont été montrées en ischémie cérébrale (9, 14, 19, 22) et traumatismes de la moelle épinière (20); dans des modèles de maladies de Parkinson et d'Huntington (1, 2, 4, 5, 16). En essais cliniques, le riluzole est, à ce jour la seule molécule efficace dans le traitement,de la sclérose latérale amyotrophique (SLA) (3, 11) et est commercialisé sous le nom de Rilutek® par la Société Rhône Poulenc France.

La mise en évidence des nouvelles propriétés du riluzole selon la présente invention est fondée sur l'étude *in vivo* de l'effet neuroprotecteur possible du riluzole dans un modèle d'ischémie rétinienne induite par augmentation de la pression intraoculaire. L'outil utilisé pour l'évaluation de la fonctionnalité de la rétine est l'électrorétinographie.

Les résultats rapportés ci-après ont permis de montrer l'effet neuroprotecteur d'un inhibiteur de la libération du glutamate et de manière surprenante l'effet neuroprotecteur du riluzole et permettent donc l'application de ce composé, de ses dérivés actifs en neuroprotection, ou de leurs sels pharmaceutiquement acceptables, à la préparation de médicaments destinés au traitement des maladies associées à l'ischémie rétinienne.

CHEW S.J. ET AL ("Neuroprotection: The next breakthrough in glaucoma ? Proceedings of The Third Annual Optic Nerve Rescue and Restoration Think Tank." JOURNAL OF GLAUCOMA, (1997) 6/4 (263-266).) et DREYER E B ("Intelligent selection of glutamate antagonists for the management of glaucoma." ANNUAL MEETING OF THE ASSOCIATION FOR RESEARCH IN VISION AND OPHTHALMOLOGY, PARTS 1-2, FORT LAUDERDALE, FLORIDA, USA, MAY 11-16, 1997. INVESTIGATIVE OPHTHALMOLOGY & VISUAL SCIENCE 38 (4 PART 1-2). 1997. S221.) ne font que suggérer que le riluzole, parmi une liste de principes actifs potentiels, pourrait avoir un pouvoir thérapeutique contre le glaucome ou l'ischémie rétinienne.

Les sels du riluzol décrits dans les brevets cités précédemment peuvent être utilisés dans le cadre de la présente invention. L'homme du métier est à même de choisir le cas échéant le plus adapté à la nouvelle application selon l'invention. Comme sels pharmaceutiquement acceptables on peut citer notamment les sels d'addition avec les acides minéraux tels que chlorhydrate, sulfate, nitrate, phosphate ou organiques tels que acétate, propionate, succinate, oxalate, benzoate, fumarate, maléate, méthanesulfonate, isétionate, etc..., ou des dérivés de substitution de ceux-ci.

Les médicaments selon l'invention sont constitués par au moins le riluzol sous forme libre ou sous forme d'un sel d'addition avec un acide pharmaceutiquement acceptable, à l'état pur ou sous forme d'une composition dans laquelle il est associé à tout autre produit pharmaceutiquement compatible. Les médicaments selon l'invention peuvent être employés par voie orale, parentérale, rectale ou topique.

A titre de compositions solides pour administration orale, on peut utiliser des comprimés, des pillules, des poudres, etc... où le riluzole est mélangé à un ou plusieurs diluants inertes classiquement utilisés, et éventuellement à d'autres substances, tels que par exemple un lubrifiant, un colorant, un enrobage etc....

A titre de compositions liquides pour administration orale ou oculaire, on peut utiliser des suspensions, des solutions, des suspensions, des émulsions, des sirops pharmaceutiquement acceptables contenant des diluants inertes classiquement utilisés, et éventuellement d'autres substances comme des produits mouillants, édulcorants, épaississant, etc....

Les compositions stériles pour administration parentérale peuvent être des solutions aqueuses ou non, des suspension ou des émulsions. Comme solvant ou véhicule, on peut employer l'eau, le propylèneglycol, des huiles végétales ou d'autres solvants organiques convenables. Ces compositions peuvent également contenir des adjuvants, comme des agents mouillants, isotonisants, émulsifiants, etc....

Les compositions pour l'administration topiques peuvent être par exemple des crèmes, lotions, collutoires, goutes nasale ou oculaires ou aérosol.

Comme cela appraît des expériences rapportés ci-après, on préfère plus particulièrement une application locale dans l'oeil de la composition de l'invention.

Les doses sont fonction de la voie d'administration utilisée. Elles sont généralement comprises entre 50 et 400 mg par jour par voie orale avec des doses unitaires de l'ordre de 25 à 200 mg de substance active.

L'application locale dans l'oeil constitue un mode d'utilisation avantageux du riluzole dans l'application selon l'invention. Ainsi, on envisage tout particulièrement une administration en collyre. L'extrapolation des résultats chez le lapin permettent d'envisager pour l'homme l'utilisation d'une solution dosée à environ 10⁻⁶ M/l à utiliser avantageusement 2 fois par jour, à raison d'une goutte d'environ 50 microlitres par oeil.

L'invention concerne aussi le procédé de préparation des médicaments selon l'invention consistant à mélanger le riluzole ou son dérivé, ou leurs sels pharmaceutiquement acceptables, avec un ou plusieurs diluants et/ou adjuvants compatibles et pharmaceutiquement acceptables.

D'autres caractéristiuqes et avantages de l'invention apparaîtront dans les exemples qui suivent.

### I - MÉTHODES.

### 1/ Animaux.

Les rats pigmentés Brown-Norway (élevage Charles River), de 250 à 300 g, sont choisis pour leur teneur oculaire importante en mélanine, ce qui permet une meilleure efficacité des essais thérapeutiques.

### 2/ Modèle d'ischémie par augmentation de la pression oculaire.

Les rats sont anesthésiés avec une injection intrapéritonéale de 6% pentobarbital (100 ml/100 g). Après l'instillation d'une goutte d'oxybuprocaïne (Laboratoire Chauvin) pour dilater la pupille et permettre ainsi le contrôle du fond d'oeil par ophtalmoscopie directe, une aiguille héparinée de 30 Gauge est placée dans la chambre antérieure de l'oeil au niveau du limbe. Elle est reliée à un réservoir contenant une solution saline isotonique (Hank's Balanced Salt Solution), colonne d'eau, qui par élévation, permet de réaliser une hyperpression oculaire d'environ 130 mm Hg. Le temps d'ischémie réalisé est de 30 minutes. Les temps de reperfusion choisis sont de 2 et 7 jours.

### 3/ Traitements des animaux.

Dans une première phase de l'expérimentation, le riluzole (8 mg/kg, Research Biochemicals International), d'abord dissous dans 0,1 N d'acide chlorhydrique, puis dilué dans du sérum physiologique, est injecté en intrapéritonéal 30 minutes avant (IP-pré-traitement) ou 30 minutes après l'ischémie (IP-post-traitement) et une fois quotidiennement jusqu'à la fin de l'expérimentation.

Dans une seconde phase de l'expérimentation, une solution de 0.02% de riluzole (20 µl), diluée dans du "Hank's Balanced Salt Solution" est appliquée localement dans l'oeil 30 minutes avant (Topical-pre-traitement) ou immédiatement après l'ischémie (Topical-post-traitement) et deux fois quotidiennement jusqu'à la fin de l'expérimentation.

### 4/ Procédure d'électrorétinogranhie.

### a) Principe.

A l'état de repos, l'oeil présente une différence de potentiel permanente dite "potentiel cornéo-rétinien" entre la cornée positive et le fond du globe oculaire négatif. L'électrorétinogramme (ERG) est le recueil de ce potentiel d'action à la surface de l'oeil. Plusieurs cellules participent à son élaboration. Il s'agit donc d'une réponse électrique globale. Le signal recueilli, traité et amplifié, peut se décomposer en 2 ondes successives:

L'onde "a" de polarité négative générée par les photorécepteurs (hyperpolarisation membranaire par modification des molécules de pigments par l'effet d'un photon entraînant un déséquilibre ionique).

L'onde "b" de polarité positive qui est essentiellement générée par les cellules de Müller (par dépolarisation).

L'onde "c" de polarité positive, très lente, apparaissant tardivement, générée en partie par l'épithélium pigmentaire en réponse à une stimulation par des bâtonnets.

L'ischémie rétinienne entraîne des perturbations des échanges ioniques cellulaires et donc de la transduction du signal. Si l'ischémie est importante et se prolonge, une perte de l'activité électrique se produit, pouvant devenir irréversible et conduire à la cécité.

### b) Technique.

Les animaux sont adaptés à l'obscurité 4 heures avant l'expérimentation. L'électrorétinographie est réalisée dans le noir sur le rat anesthésié , et après dilatation des pupilles par instillation d'une goutte de mydriaticum (0,5% tropicamide, Chibret laboratory). Une lentille de contact contenant un anneau en chlorure d'argent est placée sur la cornée (au préalable anesthésiée localement par une goutte d'oxybuprocaÏne-hydrocloride à 0,04%). L'électrode de référence est constituée d'une aiguille en argent-chlorure d'argent insérée dans l'oreille de l'animal. Une mesure électrorétinographique de base est prise avant la réalisation de l'ischémie rétinienne.

Les stimuli lumineux sont produits par un stroboscope placé à 20 cm de l'oeil, dans l'axe visuel. Le flash de lumière blanche a une durée de 10 µsecondes et une intensité de 5x10⁻⁴ cd/m² (cd est le symbole de candela, unité d'intensité lumineuse du système international).

Après stimulation par un flash lumineux, le signal électrique est recueilli par un amplificateur différentiel (gain X 1000, 0,1 - 500 Hz) visualisé sur un oscilloscope analogique numérique et transmis à un micro-ordinateur IBM. Un logiciel permet l'acquisition des données, ainsi que la détermination des amplitudes et les temps de latence des différentes ondes de l'électrorétinogramme (ERG). Les ERGs sont enregistrés à 4 minutes d'intervalle avant l'ischémie, pendant et 2 et 7 jours après l'ischémie. Six rats sont étudiés pour chaque temps de reperfusion analysé. Le paramètre utilisé pour évaluer l'ERG est l'amplitude de l'onde, mesurée en µV de la ligne de base au creux le plus bas du tracé (pour l'onde "a") et de ce creux au pic le plus haut (pour l'onde "b"). Quinze réponses consécutives sont enregistrées et les valeurs moyennes calculées et analysées statistiquement avec le test de "Mann Witney U", pour chaque groupe de rats. Les valeurs des ondes "a" et "b" dé l'ERG sont exprimées en pourcentage de la valeur initiale.

### II - HISTOPATHOLOGIE.

### a) Coloration argyrophyle à imprégnation d'agent.

Cette coloration spécifique a pour but de mettre en évidence la mort neuronale par nécrose. Elle s'effectue sur des coupes de rétine de 7 µm incluse dans la parafine selon un protocole standard décrit par Gallyas et coll. (23). L'analyse histologiue s'effectue une semaine après l'ischémie.

### b) Test d'apoptose par la méthode TUNEL (TdT-mediated dUTP nick end labelling).

La technique de détection et de quantification de l'apoptose, encore appelée mort cellulaire programmée est basée sur la fragmentation de l'ADN. Elle s'effectue selon la méthode de Gravrieli et coll. (24), à l'aide du Kit "In Situ Cell Death Detection", commercialisé par Boehringer Mannheim.

### III - RÉSULTATS.

### 1) Electorétinoaraphie.

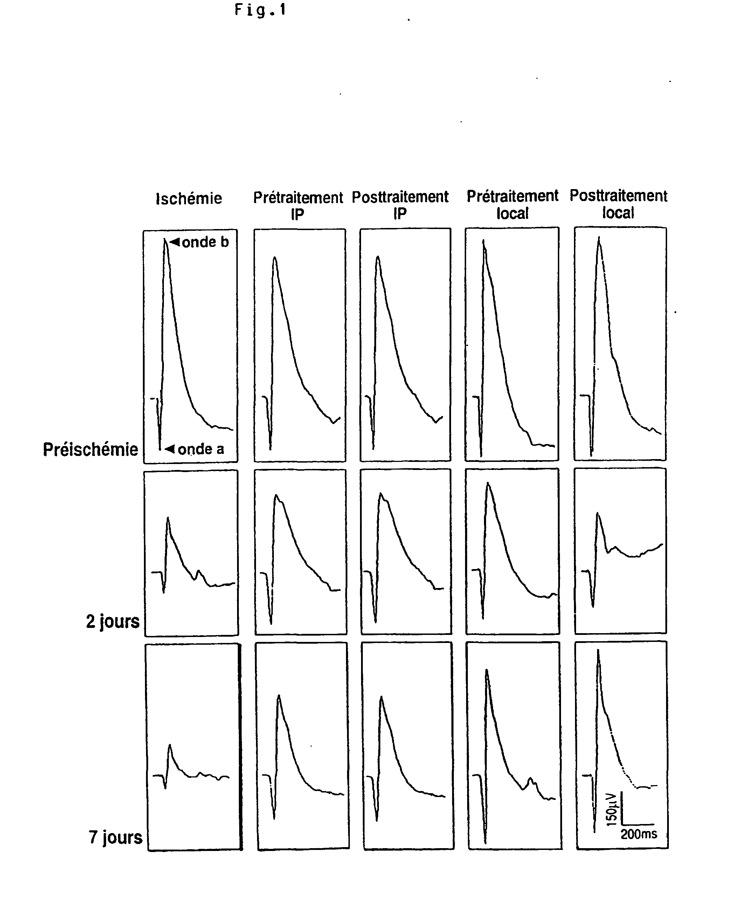

La figure 1 représente les électrorétinogrammes (ERGs) obtenus à partir de 6 rats "Témoins" et "Traités" par le riluzole enregistrés après 30 min d'ischémie induite par pression intraoculaire. Les ERGs sont enregistrés avant l'ischémie et après 2 et 7 jours de reperfusion. Le traitement par le riluzole est indiqué comme "Pré- et post-traitement IP" et "Pré- et post-traitement local. Moyenne de 15 essais consécutifs en réponse à la stimulation.

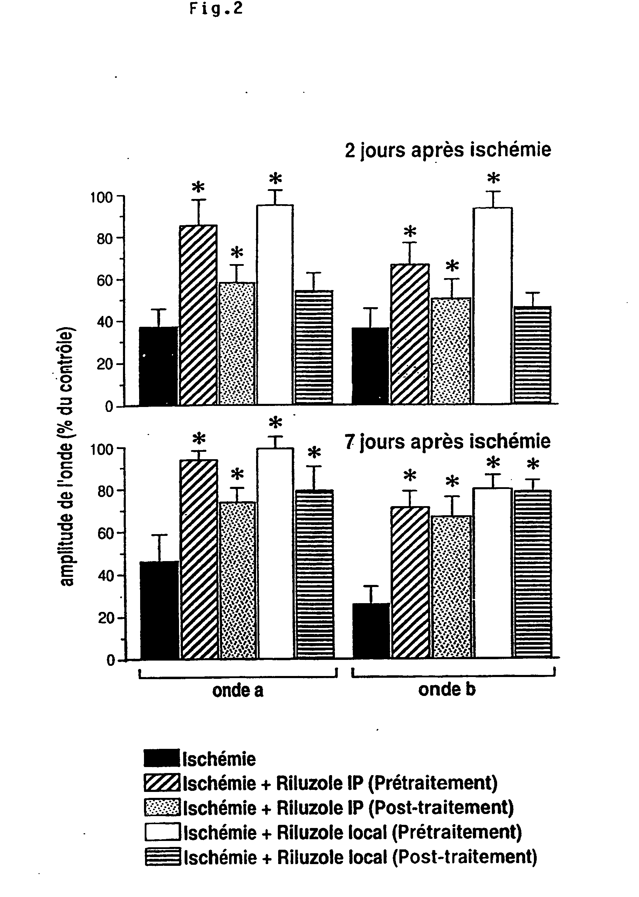

La figure 2 représente l'effet de l'ischémie avec des temps de reperfusion de 2 et 7 jours sur l'amplitude des ondes "a" et "b" d' ERG chez des rats "Témoins" et "Traités" par le riluzole (IP = injection intrapéritoneale ou Topical = instillation locale 30 min avant (pré-traitement) ou 30 min après ischémie (post-traitement). Le riluzole est administré quotidiennement durant le temps de reperfusion. Les amplitudes des ondes "a" et "b" sont exprimées en pourcentage des valeurs pré-ischémiques respectives (% of control). Les différences statistiquement significatives entre rats "Témoins" et rats "Traités" sont indiquées par des astérisques (P<0.01 *versus* Témoin, n = 6, Mann Witney U test).

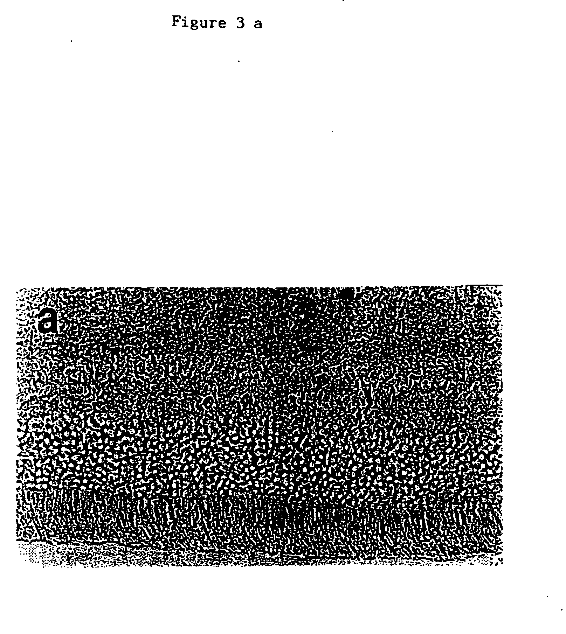

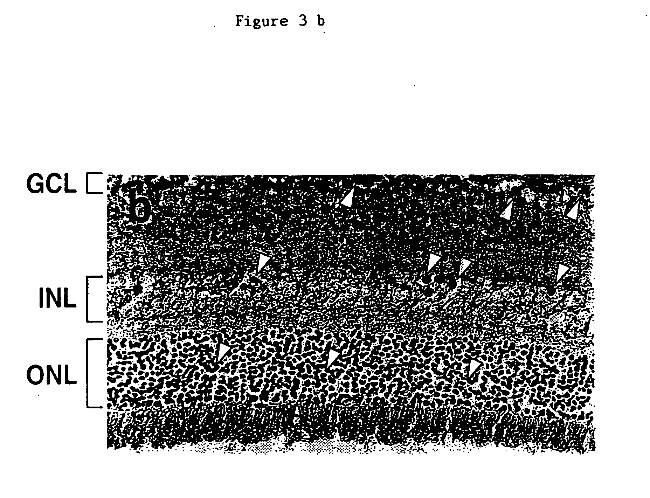

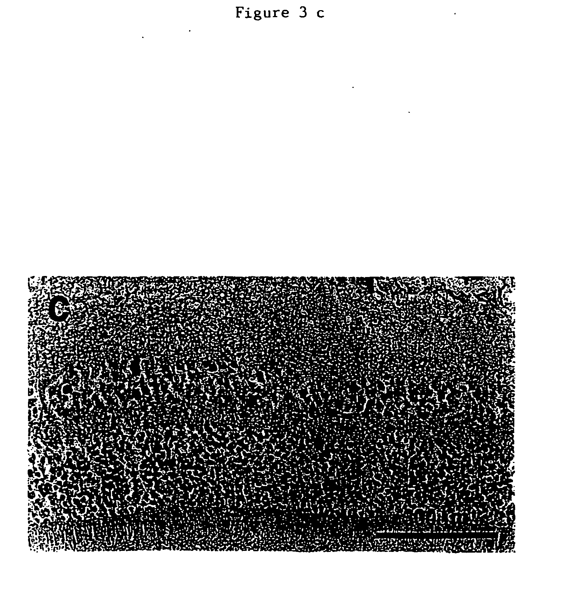

La figure 3 représente des photomicrographies montrant la morphologie de la rétine colorée par la méthode à imprégnation argentique chez des rats "Témoins" (a), des rats ischémiés non-traités (b) et des rats ischémiés traités au riluzole en application locale (c), observés après 7 jours de reperfusion. Les précipités de grains d'argent indiqués par des flèches apparaissent dans la couche ganglionnaire (GCL) et les couches nucléaires interne (INL) et externe (ONL) de la rétine endommagée.

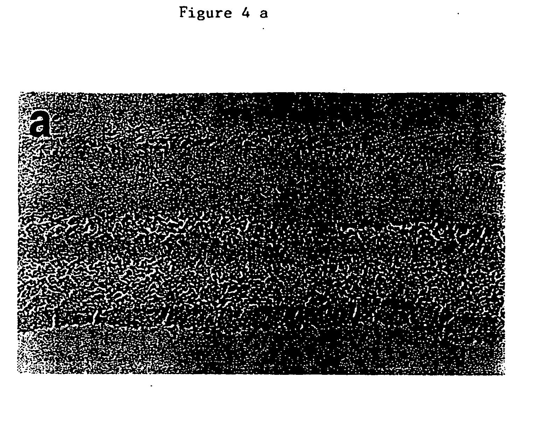

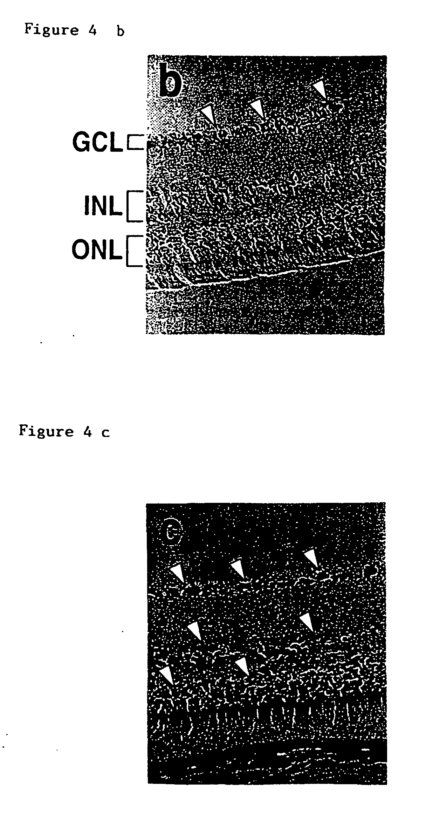

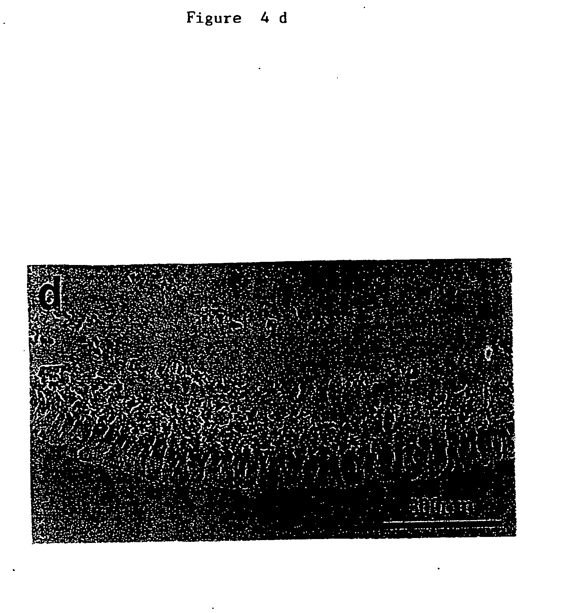

La figure 4 représente des photomicrographies montrant les cellules apoptiques détectées par la méthode TUNEL dans des coupes de rétine de rat après 30 minutes d'ischémie-reperfusion et l'effet du riluzole en application locale. Dans la rétine de rat "Témoin" (a), il n'y a aucune cellule apoptique. Après une ischémie de 30 minutes induite par une hype-pression oculaire, le marquage spécifique des cellules apoptiques (flèches) apparait dans la couche ganglionnaire (CGL) après deux jours de reperfusion (b) et additionnellement dans les couches nucléaires internes (INL) et externe (ONL) après 4 jours de reperfusion (c). Das la rétine de rat traité au riluzole (d), il y a absence totale de cellules apoptotiques après 30 minutes d'ischémie et 4 jours de reperfusion.

La Figure 1 compare les profils des ERGs juste avant et 2 jours et 7 jours après l'ischémie chez des rats "Témoins" et des rats "Traités". Les ERGs contrôles des différents groupes de rats sont stables et non significativement différents des valeurs de niveau de base. Durant l'ischémie, induite par hyper-pression intraoculaire pendant 30 min , le tracé des ondes "a" et "b" devient plat (non représenté). Durant la reperfusion, en absence de traitement par le riluzole, les ondes "a" et "b" récupèrent lentement (Fig. 1 et 2). Deux jours après l'ischémie, l'amplitude des ondes "a" et "b" est respectivement de 38% et 26% de la valeur initiale. Après un temps de reperfusion d'une semaine, l'amplitude des ondes "a" et "b" se stabilise, respectivement à 27% et 37% des valeurs initiales.

Les ERGs enregistrés chez des rats non-ischémiés mais traités au riluzole restent inchangés au cours du temps et l'amplitude des ondes "a" et "b" n'est pas affectée par le traitement (Fig. 1). Dans tous les groupes de rats traités, le riluzole empêche l'altération des ondes "a" et "b" induite par l'ischémie (Fig. 1 et 2). Sept jours après l'ischémie, la récupération de l'amplitude des ondes "a" et "b" est approximativement de 40% des valeurs initiales chez les rats "Témoins", alors qu'elle se situe entre 65% et 95% chez les rats "Traités". Le type d'administration du riluzole (injection intrapéritonéale ou traitement local) et le timing du traitement (avant ou après ischémie) ne semblent pas avoir d'incidence majeure sur la récupération de l'onde "b" après un temps long de reperfusion (7 jours). Dans tous les cas, l'amplitude de l'onde "b" est d'environ 75% de la valeur contrôle. A des temps plus courts de reperfusion (2 jours après l'ischémie), le riluzole en application locale avant l'ischémie produit clairement la protection la plus efficace (Fig. 2). La récupération de l'onde "a" est aussi excellente après une semaine de reperfusion chez les rats traités au riluzole. La meilleure récupération de l'amplitude de l'onde "a" est obtenue quand le riluzole est administré avant l'ischémie, à la fois en traitements intrapéritonéal et local et après 2 jours ou 7 jours de reperfusion (Fig. 2).

### 2) Histopatologie.

La mort neuronale induite par ischémie se produit par nécrose et apoptose. La nécrose est mise en évidence par la coloration argyrophyle, sous forme de précipités de grains d'argent visibles au niveau du noyau des cellules. Aucune coloration n'est visualisée sur la rétine des rats "Témoins" (Figure 3a). Par contre, la figure 3b révèle que les cellules rétiniennes sont sévèrement endommagées par l'ischémie. Des précipités de grains d'argent apparaissent dans la couche des cellules ganglionnaires (GCL) et les couches nucléaires internes (INL) et externe (ONL) après ischémie et 7 jours de reperfusion. La figure 3c montre le spectaculaire effet du riluzole. Un traitement avant et/ou après ischémie en injection intrapéritonéale ou en application locale protège totalement la rétine de l'ischémie. La figure 3c révèle l'absence de précipités de grains d'argent après une application locale de riluzole 30 minutes après l'ischémie et 7 jours de reperfusion dans les cellules des 3 couches rétiniennes connues pour être lésées après une iscémie.

La mise en évidence d'un processus d'apoptose est réalisée gràce à la méthode de fragmentation de l'ADN dite méthode "TUNEL". Les cellules apoptotiques sont visualisées sous forme d'une condensation nucléaire de coloration rose foncé. La rétine des rats "Témoins" ne présente aucune coloration (Figure 4a). Les coupes rétiniennes des rats "Ischémiés" montrent la présence d'apoptose au niveau de la couche ganglionnaire (GCL) après ischémie et à 2 jours de reperfusion (Figure 4b) et au niveau des couches nucléaires internes (INL) et externe (ONL) à 4 jours de reperfusion (Figure 4c). Cette analyse de l'apoptose révèle de nouveau la neuroprotection induite par le riluzole. L'administration systémique et locale du riluzole réduit considérablement la mort par apoptose. La figure 4d montre qu'une application locale de riluzole 30 minutes après l'ischémie résulte de la disparition pratiquement totale des cellules apoptotiques dans la couche ganglionnaire et les couches des photorécepteurs 4 jours après l'ischémie.

### III - CONCLUSION.

L'ischémie induite par augmentation de la pression oculaire est le modèle le plus fréquemment utilisé pour étudier à la fois les mécanismes et les thérapies potentielles de l'ischémie rétinienne. Le degré de lésion de la rétine est dépendante de l'espèce animale, de la durée et de l'intensité de la pression oculaire imposée. Dans .la présente étude, ce modèle est appliqué au rat avec une pression intraoculaire de 130 mm Hg pendant 30 minutes pour produire l'ischémie. La réponse électrique de la rétine est sévèrement altérée et les mécanismes moléculaires déjà engagés, pour aboutir à la mort des cellules retiniennes. Les travaux réalisés dans le cadre de la présente invention démontrent pour la première fois le spectaculaire effet neuroprotecteur du riluzole contre l'ischémie rétinienne par l'étude des ERGs pour évaluer les dommages de la rétine. A ce jour, il n'existait aucun traitement efficace contre la dégénérescence de la rétine induite par ischémie. Les présents travaux montrent que le riluzole, à la fois en administration systémique ou locale améliore spectaculairement la récupération des ondes "a" et "b" de l'ERG comparé à un rat non traité. Après un pré- et même un post-traitement au riluzole appliqué localement, les ondes "a" et "b" sont proches de l'amplitude de celles d'animaux "Témoins" après une semaine de reperfusion.

La mort neuronale induite par ischémie se produit par apoptose et/ou par nécrose, deux types de mort cellulaire bien disticts, qui impliquent des mécanismes soit actif soit passif. Dans ces travaux, les neurones apoptotiques sont détectés par la méthode TUNEL, qui marque la fragmentation del'ADN dans les noyaux cellulaires et les neurones nécrosés sont visualisés par la coloration à imprégnation argentique, oùles précipités de grains d'argent apparaissent dans les noyauxdes neurones en dégénérescence. D'abondantes cellules apoptotiques et des neurones argyrophyles sont visibles dans les couches GCL, INL et ONL des rétines de rats ayant subi une hyper-pression oculaire. Le riluzole, très clairement, empêche à la fois la nécrose et les réactions de gliose et réduit considérablement le phénomène d'apoptose. L'application locale par rapport à une injection systémique (intrapéritonéale) est le moyen le plus efficace pour protéger la rétine. Le pré-traitement du riluzole en application locale (avant ischémie) induit un effet neuroprotecteur à des temps plus courts de reperfusion, tandis qu'un post-traitement en application locale est plus efficace à des temps plus longs de reperfusion. Ces résultats reportés ici (protection et traitement local) désignent le riluzole comme une importante molécule potentielle en ophtalmologie. Ces effets s'appliquent à d'autres pathologies rétiniennes importantes, telles que le glaucome, la rétinopathie pigmentaire et la dégénérescence maculaire liée à l'âge (DMLA) et que dans toutes ces pathologies, le riluzole se comporte comme un neuroprotecteur.

### RÉFÉRENCES

1/ Barneoud, P., M. Mazadier, J. M. Miquet, S. Parmentier, P. Dubedat, A. Doble and A. Boireau. (1996). "Neuroprotective effects of riluzole on a model of Parkinson's disease in the rat." Neuroscience. 74: 971-983.
2/ Benazzouz, A., T. Boraud, P. Dubedat, A. Boireau, J. M. Stutzmann and C. Gross. (1995). "Riluzole prevents MPTP-induced parkinsonism in the rhesus monkey: a pilot study." Eur. J. Pharmacol. 284: 299-307.
3/ Bensimon, G., L. Lacomblez and V. Meininger. (1994). "A controlled trial of riluzole in amyotrophic lateral sclerosis." N. Engl. J. Med. 330: 585-591.
4/ Boireau, A., P. Dubedat, F. Bordier, C. Peny, J.-M. Miquet, G. Durand, M. Meunier and A. Doble. (1994a). "Riluzole and experimental parkinsonism: antagonism of MPTP-induced decrease in central dopamine levels in mice." Neuroreport. 5: 2657-2660.
5/ Boireau, A., P. Dubedat, F. Bordier, C. Peny, J.-M. Miquet, G. Durand, M. Meunier and A. Doble. (1994b). "Riluzole and experimental parkinsonism: partial antagonism of MPTP-induced increase in striatal extracellular dopamine in rats *in vivo."* Neuroreport. 5: 2157-2160.
6/ Cheramy, A., L. Barbeiro, G. Godeheu and J. Glowinski. (1992). "Riluzole inhibits the release of glutamate in the caudate nucleus of the cat in vivo." Neurosci. Lett. 147: 209-212.
7/ Choi, D. W. (1938). "Glutamate neurotoxicity and diseases of the nervous sytem." Neuron. 1: 623-634.
8/ Dreyer, E. B., D. Zurakowski, R. A. Schumer, S. M. Podos and S. A. Lipton. (1996). "Elevated glutamate in the vitreous body of humans and monkeys with glaucoma." Arch. Ophthalmol. 114: 299-305.
9/ Heurteaux, C., I. Lauritzen, C. Widmann and M. Lazdunski. (1994). "Glutamate-induced overexpression of NMDA receptor messenger RNAs and protein triggered by activation of AMPA/kainate receptors in rat hippocampus following forebrain ischemia." Brain Res. 659: 67-74.
10/ Hubert, J. P. and A. Doble. (1989). "Ibotenic acid stimulates D-(³H) aspartate release from cultured cerebellar granule cells." Neurosci. Lett. 96: 345-350.
11/ Lacomblez, L., G. Bensimon, P. N. Leigh, P. Guillet and V. Meininger. (1996). "For the ALS/Riluzole study group II. Dose-ranging study of riluzole in amyotrophic lateral sclerosis." Lancet. 347: 1425-1431.
12/ Louzada-Junior, P., J. J. Dias, W. F. Santos, L. J. J, H. F. Bradford and J. Coutinho-Netto. (1992). "Glutamate release in experimental ischaemia of the retina : an approach using microdialysis." J. Neurochem. 59: 358-363.
13/ Lucas, D. R. and J. P. Newhouse. (1957). "The toxic effect of sodium L-glutamate on the inner layers of the retina." Am. Med. Arch. Ophtalmol. 53: 193-201.
14/ Malgouris, C., F. Bardot, M. Daniel, F. Pellis, J. Rataud, A. Uzan, J.-C. Blanchard and P. M. Laduron. (1989). "Riluzole, a novel antiglutamate prevents memory loss and hippocampal neuronal damage in ischemic gerbils." J. Neurosci. 9: 3720-3727.
15/ Martin, D., M. A. Thompson and J. V. Nadler. (1993). "The neuroprotective agent riluzole inhibits release of glutamate and aspartate from slices of hippocampal area." Eur. J. Pharmacol. 250: 473-476.
16/ Mary, V., F. Wahl and J. M. Stutzmann. (1995). "Effect of riluzole on quinolinate-induced neuronal damage in rats: comparison with blockers of glutatergic neurotransmission." Neurosci. Lett. 201: 92-96.
17/ Massey, S. C. (1990). "Cell types using glutamate as a neurotransmitter in the vertebrate retina". In *Retinal Research.* Oxford. New York, Pergamon Press.
18/ Olney, J. W. (1986). "Inciting excitotoxic cytocide among central neurons." Adv. Exp. Med. Biol. 203: 631-645.
19/ Pratt, J., J. Rataud, F. Bardot, M. Roux, J. C. Blanchard, P. M. Laduron and J. M. Stutzmann. (1992). "Neuroprotective actions of Riluzole in rodent models of global and focal cerebral ischemia." Neurosci. Lett. 140: 225-230.
20/ Stutzmann, J. M., J. Pratt, T. Boraud and C. Gross. (1996). "The effect of riluzole on post traumatic spinal cord injury in the rat." Neuroreport. 7: 387-392.
21/ Vorwerk, C. K., S. A. Lipton, D. Zurakowski, B. T. Hyman, B. A. Sabel and E. B. Dreyer. (1996). "Chronic low-dose glutamate is toxic to retinal ganglion cells. Toxicity blocked by Memantine." Invest. Ophthalmol. Vis. Sci. 37: 1618-1624.
22/ Wahl, F., M. Allix, M. Plokine and R. G. Boulu. (1993). "Effect of riluzole on focal cerebral ischemia in rats." Eur. J. Pharmacol. 230: 209-214.
23/ Gallyas, F., Güldner, F.H., Zoltay, G. and Wolff, J.R. (1990) Golgi-like demonstration of "dark" neurons with an argynophyl III method for experimental neuropathology. *Acta neuropathol*., 79, 620-628.
24/ Gavrieli, Y., Sherman, Y. and Ben-Sason, S.A. (1992) Identification of programmated death cell *in situ via* specific labeling of nuclear DNA fragmentation. *J.Cell. Biol.,* 11, 493-501.

## Revendications

1. Utilisation du riluzole ou de ses dérivés actifs en neuroprotection ou des sels pharmaceutiquement acceptables de ces composés, pour la préparation de médicaments destinés au traitement des maladies associées à une ischémie rétinienne.

2. Utilisation selon la revendication 1, **caractérisée en ce que** lesdits médicaments sont destinés au traitement des occlusions artérielles ou veineuses de la rétine, des contusions oculaires, de la dégénérescence maculaire liée à l'âge, du glaucome, de la rétinopathie diabétique, de la rétinopathie du prématuré, des maladies inflammatoires et des hémopathies.

3. Utilisation selon l'une quelconque des revendications 1 ou 2, **caractérisée en ce que** lesdits médicaments sont destinés à une application locale dans l'oeil.

## Patentansprüche

1. Gebrauch von Riluzol oder seinen aktiven Derivaten im Neuroschutz oder den pharmazeutisch akzeptierbaren Salzen dieser Zusammensetzungen, für die Vorbereitung von Medikamenten bestimmt zur Behandlung der Krankheiten verbunden mit einer Ischämie der Netzhaut.

2. Gebrauch nach Anspruch 1, charakterisiert **dadurch** dass besagte Medikamente bestimmt sind zur Behandlung der arteriellen oder venösen Okklusion der Netzhaut, der Augenquetschungen, der altersbedingten makulären Degeneration, des grünen Stars, der diabetischen Retinopathie, der Retinotherapie der Frühgeburt, der entzündlichen Krankheiten und der Blutkrankheiten.

3. Gebrauch nach einem der Ansprüche 1 oder 2, charakterisiert **dadurch** dass besagte Medikamente bestimmt sind zur lokalen Behandlung im Auge.

## Claims

1. Use of riluzole or of its derivatives active in neuroprotection or pharmaceutically acceptable salts of these compounds, for preparing drugs intended to treat diseases associated with retinal ischemia.

2. The use according to claim 1, **characterized in that** said drugs are intended to treat arterial or venous occlusions of the retina, ocular contusions, age-related macular degeneration, glaucoma, diabetic retinopathy, premature infant retinopathy, inflammatory diseases and haemopathies.

3. The use according to any claims 1 or 2, **characterized in that** said drugs are intended for a local application in the eye.
